(19) Europäisches Patentamt
European Patent Office
Office européen des brevets

(11) **EP 4 696 372 A1**

(12) **EUROPEAN PATENT APPLICATION**

(43) Date of publication:
**18.02.2026 Bulletin 2026/08**

(21) Application number: **24194359.6**

(22) Date of filing: **13.08.2024**

(51) International Patent Classification (IPC):
***A61N 5/10*** (2006.01)

(52) Cooperative Patent Classification (CPC):
**A61N 5/103;** A61N 2005/1087

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers International AG**
**6312 Steinhausen (CH)**

(72) Inventors:
• **FALKOVSKIY, Pavel**
  **00420 Helsinki (FI)**
• **BAARDSEN, Gustav**
  **00640 Helsinki (FI)**

(74) Representative: **Mathisen & Macara LLP**
**Charta House**
**30-38 Church Street**
**Staines-upon-Thames TW18 4EP (GB)**

(54) **ENERGY LAYER SPACING IN PARTICLE THERAPY TREATMENT PLANS**

(57) There is provided a method (600) of computer-assisted particle therapy treatment planning comprising generating a treatment plan that uses non-uniform energy layer spacing (550). The treatment plan may be optimised using an objective function that permits non-uniform energy layer spacing (550) in the generated treatment plan. The objective function may comprise a cost function with terms that penalise deviation from clinical goals, and said optimisation may comprise minimising the cost function; and the objective function may contain a penalty term that guides the optimisation of the treatment plan to provide non-uniform energy layer (550) spacing in the generated treatment plan.

FIG. 5

## Description

### TECHNICAL FIELD

This invention relates to particle therapy treatment planning, and in particular, to methods and systems for generating particle therapy treatment plans that use non-uniform energy layer spacing.

### BACKGROUND

Particle therapy with protons or other charged particles is a type of radiotherapy that uses an external beam to provide targeted ionising radiation to a tumour. Protons or other charged particles are sent to an accelerator to bring the particles' energy to a predetermined value. The protons or other charged particles then move through a beam-transport system, where magnets are used to shape, focus and/or direct the proton beam or other particle beam as necessary. The process of determining and controlling the path of the proton beam through the beam transport system from the accelerator to the patient is referred to as "beamline" calculations.

Typically, particle radiation therapy systems deposit energy in "spots" along the path of the beam to a target tumour. Spots are grouped into "energy layers" wherein spots in an energy layer have the same energy. Here, reference is made to the well-known concept of a Bragg-peak in particle therapy, where the particles in a particle beam deposit a substantial proportion of their energy at the Bragg peak, just before the end of the particle's trajectory. Each energy layer corresponds to particle beams of a particular energy and a particular Bragg peak depth. (In the present specification, references to "layers" are to be taken as references to "energy layers", unless the context requires a different meaning.)

Radiation treatment plans can be optimised according to given dose objectives for the target volume (i.e., the tumour) and organs at risk (OARs, i.e., healthy tissue) using commercially available treatment planning systems. Dose distributions are calculated using beam characteristics and a machine specific dose calibration. The treatment plan can be optimised for efficiency and this optimisation may employ an iterative trial-and-error modification of several treatment plan parameters (e.g., energy layer distance, spot size or spot spacing) in a multiobjective optimisation process.

However, particle therapy planning using existing techniques is constrained by the delivery of spots in fixed, discrete, uniformly spaced energy layers. For example, to deliver dose to a 3D volume, multiple discrete uniformly spaced beam energy layers are generally used. Traditionally, the discretisation scheme including the selection of layer energies and the selection of (uniform) energy layer spacing are decided based on patient geometry and hardware constraints, and the treatment planning optimisation is limited by these constraints.

That is, in the prior art, the problem of optimising treatment plans for particle therapy such as Intensity Modulated Proton Therapy (IMPT) has been addressed using a constant (uniform) and predetermined value for energy layer spacing. The value is manually chosen as a compromise between two critical variables:

- *Treatment Quality*: Smaller energy layer spacing allows for more accurate, precise and finer-level targeting of the tumour, leading to better treatment outcomes. However, this requires more energy layers, which increases the complexity and time of the treatment delivery.

- *Delivery Time / plan complexity*: Larger energy layer spacing reduces the number of energy layers and thus reduces the time required for treatment delivery and also reduces the complexity of the treatment plan. However, this can compromise the accuracy and efficacy of the treatment, potentially affecting the therapeutic outcome.

In particular, there is a need to balance (i) treatment quality and (ii) delivery time / complexity in particle therapy treatment plans. As noted, traditional methods rely on fixed energy layer spacing, where the distance between each layer is uniform, constant and predetermined. This can lead to reduced treatment accuracy (due to larger energy layer spacing) or to increased treatment complexity / delivery times (due to finer layer spacing).

### SUMMARY OF THE INVENTION

In accordance with a first aspect of the present invention, there is provided a method of computer-assisted particle therapy treatment planning as defined by claim 1.

In accordance with a second aspect of the present invention, there is provided a radiation treatment planning computer system as defined by claim 14.

In accordance with a third aspect of the present invention, there is provided a computer program product as defined by claim 15.

Optional features are defined by dependent claims.

The first aspect of the invention comprises a method of computer-assisted particle therapy treatment planning comprising generating a treatment plan that uses non-uniform energy layer spacing.

The method may comprise optimising the treatment plan using an objective function that permits non-uniform energy layer spacing in the generated treatment plan.

The objective function may be configured to guide the optimisation process to produce treatment plans utilising non-uniform energy layer spacing.

The objective function may comprise a cost function with terms that penalise deviation from clinical goals, wherein said optimisation comprises minimising the cost function; and wherein the objective function may further contain a penalty term that guides the optimisation of the treatment plan to provide non-uniform energy layer spacing in the generated treatment plan.

Alternatively, the objective function may comprise a utility function with terms that reward adherence to clinical goals, and wherein said optimisation comprises maximising the utility function; and wherein the objective function may further contain a reward term that guides the optimisation to provide non-uniform energy layer spacing in the generated treatment plan.

In general in the present disclosure, a "utility function" may be used instead of a "cost function", wherein the optimiser attempts to maximise the utility of the utility function instead of minimising the cost of the cost function.

Optionally, the penalty term penalises energy layers that are closer in energy level than a threshold energy proximity. The phrase "energy proximity" as used in this specification refers to how close energy layers are in terms of their respective energy levels.

Optionally, the penalty term penalises spots in dependence on spot weight.

Optionally, the penalty term penalises spots with an increasing penalty for decreasing spot weights, and/or penalises spots with a constant penalty for spots having a weight lower than a threshold weight.

Optionally, the penalty term both (i) penalises energy layers that are closer in energy level than a threshold energy proximity and (ii) penalises spots in dependence on spot weight.

Optionally, the penalty term both (i) penalises energy layers that are closer in energy level than a threshold energy proximity and (ii) penalises spots with an increasing penalty for decreasing spot weights and/or penalises spots with a constant penalty for spots having a weight lower than a threshold weight.

Optionally, the objective function comprises a layer penalty term for each energy layer, and wherein the layer penalty term for an energy layer is non-zero only if a sum of spot weights in the energy layer is lower than a layer sum threshold. Additionally or alternatively, the layer penalty term for an energy layer may be non-zero only if a sum of squared spot weights in the energy layer is lower than a layer sum threshold. Additionally or alternatively, the layer penalty term for an energy layer may be non-zero only if a norm of the total set of spot weights in the energy layer is lower than a layer sum threshold.

The sum of all spot weights in each energy layer may be referred to as the energy layer spot weights sum. Each energy layer has a corresponding energy layer spot weights sum. The spot weight sum of the energy layer with the largest spot weights sum is referred to as the maximum spot weights sum. The layer sum threshold mentioned above may comprise a percentage of the maximum spot weights sum.

Optionally, the layer penalty term is configured so that energy layers with all spots having a weight that is below a global spot weight threshold are not penalised. Optionally, energy layers with all spots having a weight below the global spot weight threshold are removed in a post-processing step.

The method may further comprise weighting the penalty term with respect to other terms in the objective function to control an extent of non-uniformity in the energy layer spacing.

A higher weight of the penalty term increases the possibility of discarding spots to achieve non-uniform energy layer spacing. Conversely, a lower weight of the penalty term decreases the possibility of discarding spots to achieve non-uniform energy layer spacing. The weight of the penalty term may be inputted by a human user. Alternatively, the weight of the penalty term may be determined automatically so that the weight of the penalty term is a certain percentage of a weight of the rest of the cost function. When determining the weight of the penalty term automatically, the weight may be a percentage of the rest of the cost function at a specific iteration (for example at the end of an initial optimisation without the penalty term). Later in the optimisation, this percentage may vary.

Objective functions with a low weight for the penalty term would retain non-zero low-weight spots in layers where at least one spot weight is above the global spot weight threshold, to a greater extent than objective functions with a higher weight for the penalty term. Thus, by using a lower weight for the penalty term, more layers with such spots may be retained if this is required by the overall clinical objectives. By using a higher weight for the penalty term, more layers with such spots may be discarded if this is allowed by the overall clinical objectives.

Optionally, the method further comprises: optimising an initial treatment plan until convergence using uniform layer spacing, wherein optimisation of the initial treatment plan does not use a penalty term that guides the optimisation to non-uniform energy layer spacing; and iteratively applying the objective function to the initial treatment plan and to successive intermediate treatment plans to optimise and obtain a final treatment plan that uses non-uniform energy layer spacing.

One or more spot weights in the energy layers may be modified after each iteration.

The optimisation continues by modifying the spot weights in the energy layer and re-computing the cost of the objective function including the penalty term, until the cost of the objective function is minimised, or until one of one or more iteration stop conditions is met (see below).

The objective function may comprise a combination of (i) a conventional cost function and (ii) the penalty term for non-uniform energy layer spacing. The objective function may be configured to further increase the penalty if, during optimisation, the value of the conventional cost function part of the objective function increases with respect to the value of the conventional cost function prior to optimisation using the penalty term.

The penalty term may be formulated to allow the value of the cost function to increase during the iterative optimisation. Advantageously, this increases the probability of the optimisation process escaping any local minima and to eventually arrive at a global minimum.

The method may further comprise stopping the iterative optimisation when none of the energy layers with at least one spot weight above the global spot weight threshold, have a spot weight sum lower than an iteration stop spot weight sum threshold.

After the modification of the spot weights during the optimisation process using the objective function and penalty term, there will be some energy layers with all spots having weights lower than the global spot weight threshold. Layers with all spot weights lower than the global spot weight threshold may be discarded in a post-processing step. This may result in an optimal non-uniform energy layer spacing.

The method may further comprise stopping the iterative optimisation after a threshold length of time, and/or a threshold number of iterations, have lapsed since a start of the optimisation.

The method may further comprise selecting (i) the layer spacing of the initial treatment plan and (ii) a weight of the penalty term, to optimise target dose homogeneity. In particular, to achieve sufficiently homogenous dose in the target(s), it is necessary to start from a sufficiently dense grid of energy layers and not weight the penalty term too heavily.

The method may further comprise: during optimisation, storing one or more intermediate treatment plans; calculating a cost of each of the intermediate treatment plans; at the end of the optimisation, comparing each intermediate treatment plan to the final treatment plan; and selecting between the intermediate treatment plans and the final treatment plan in dependence on a plan that has a lowest cost. Optionally, the following aspects of the intermediate plans and the final treatment plan may be compared: objective function values for organs at risk and tumours, DVH curves for regions of interest, and the extent to which each intermediate treatment plan fulfils the clinical objectives or clinical goals. The treatment plan may then be selected on the basis of one or more of these comparisons.

The method may further comprise: calculating a cost of the initial treatment plan that uses uniform layer spacing; at the end of the optimisation, comparing the initial treatment plan to the final treatment plan; and selecting between the initial treatment plan and the final treatment plan in dependence on a plan that has a lowest cost. Optionally, the following aspects of the initial treatment plan and the final treatment plan may be compared: objective function values for organs at risk and tumours, DVH curves for regions of interest, and the extent to which the initial treatment plan fulfils the clinical objectives or clinical goals. The treatment plan may then be selected on the basis of one or more of these comparisons.

The particle therapy treatment plan may be optimised using multi-objective optimisation, wherein at least one objective of the multi-objective optimisation comprises minimising low-importance energy proximate layers, and wherein the selected final treatment plan is Pareto-optimal. A Pareto-optimal solution in multi-objective optimisation is where none of the objectives can be improved without a deterioration of another objective.

The penalty term may be a non-convex function with a plurality of local minima.

The method may further comprise: when a spot weight is reduced during the optimisation, increasing a spot weight of one or more corresponding spots in neighbouring layers. Advantageously, the loss of dose contribution from spots removed during the optimisation process may be compensated for by increasing the spot weights of neighbouring spots.

The method may further comprise optimising the objective function using one or more of the following optimisation techniques: simulated annealing; genetic algorithms; and the Limited-Memory Broyden-Fletcher-Goldfarb-Shanno (LBEGS) algorithm.

According to the second aspect of the invention, there is provided a radiation treatment planning computer system comprising a memory and a processor configured to perform the method as defined above.

In particular, according to the second aspect of the invention, there is provided a radiation treatment planning computer system comprising a memory and a processor configured to generate a treatment plan that uses non-uniform energy layer spacing.

According to the third aspect of the invention, there is provided a computer program product comprising a non-transitory, computer readable storage medium encoded with instructions operable for execution by a processor to perform the method as defined above.

In particular, in accordance with the third aspect of the invention, there is provided a computer program product comprising a non-transitory, computer readable storage medium encoded with instructions operable for execution by a processor to generate a treatment plan that uses non-uniform energy layer spacing.

According to a fourth aspect of the invention, there is provided a radiation treatment planning computer system comprising means to perform the method as defined above.

In particular, in accordance with the fourth aspect of the invention, there is provided a radiation treatment planning computer system comprising means for generating a treatment plan that uses non-uniform energy layer spacing.

As some embodiments of the present invention may discard or retain energy layers, they are concerned with determining the energy of the particle (e.g. proton) beam during treatment, and therefore can be seen as a form of "beamline" calculation.

Conventional methods do not typically include mechanisms specifically designed to optimise layer spacing dynamically during treatment planning, nor do they employ non-uniform energy layer spacing. Some embodiments of the present invention may result in improved treatment efficiency. By dynamically adjusting and allowing non-uniform layer spacing, some embodiments of the present invention may reduce treatment delivery time without compromising the quality and precision of the treatment.

Some embodiments of the present invention may also result in improved treatment outcomes. Compared to a uniform layer spacing that gives the same treatment time, the fine initial layer spacing and subsequent optimisation for non-uniform energy layer spacing as in some embodiments of the present invention provide selective precision which allows for more accurate targeting of the tumour, improving the Tumour Control Probability (TCP) while reducing the Normal Tissue Complication Probability (NTCP).

Further, some embodiments of the present invention provide a treatment plan that is adapted to the needs of a unique patient. As every patient geometry is unique, the treatment plan is configured to provide precise irradiation (by means of finer energy layer spacing) where clinically acceptable for the particular patient, and to provide sparser irradiation (by means of coarser energy layer spacing) where clinically acceptable for the particular patient.

Thus, the present disclosure aims to provide a radiation treatment planning method that allows for more efficient and precise targeting of tumours while minimising treatment delivery time and plan complexity.

## BRIEF DESCRIPTION OF THE DRAWINGS

For a better understanding of the present disclosure, some embodiments will now be described by way of example with reference to the accompanying drawings, in which:

Figure 1 shows a block diagram of an example of a computing system upon which some embodiments described herein may be implemented.

Figure 2 is a block diagram showing selected components of a radiation treatment system upon which some embodiments according to the present invention can be implemented.

Figure 3 illustrates elements of an exemplary radiation treatment system upon which radiation treatment plans generated according to some embodiments of the present invention may be implemented.

Figure 4 illustrates the Bragg peaks associated with various spots, showing the spot position with respect to the energy layers.

Figure 5 illustrates uniform energy layer spacing and non-uniform energy layer spacing.

Figure 6 illustrates a method of optimising a radiation treatment plan to provide non-uniform energy layer spacing.

## DETAILED DESCRIPTION

Reference will now be made in detail to several embodiments. While the subject matter will be described in conjunction with the alternative embodiments, it will be understood that they are not intended to limit the claimed subject matter to these embodiments. On the contrary, the claimed subject matter is intended to cover alternatives, modifications, and equivalents, which may be included within the scope of the claimed subject matter as defined by the appended claims.

Furthermore, in the following detailed description, numerous specific details are set forth in order to provide a thorough understanding of the claimed subject matter. However, it will be recognised by one skilled in the art that embodiments may be practised without these specific details or with equivalents thereof. In other instances, well-known methods, procedures, components, and circuits have not been described in detail as not to unnecessarily obscure aspects and features of the subject matter.

The following description is presented to enable a person skilled in the art to make and use the embodiments of this invention; it is presented in the context of a particular application and its requirements. Various modifications to the disclosed embodiments will be readily apparent to those skilled in the art, and the general principles defined herein may be applied to other embodiments and applications without departing from the scope of the present disclosure. Thus, the present invention is not limited to the embodiments shown, but is to be accorded the widest scope consistent with the principles and features disclosed herein.

Computer system

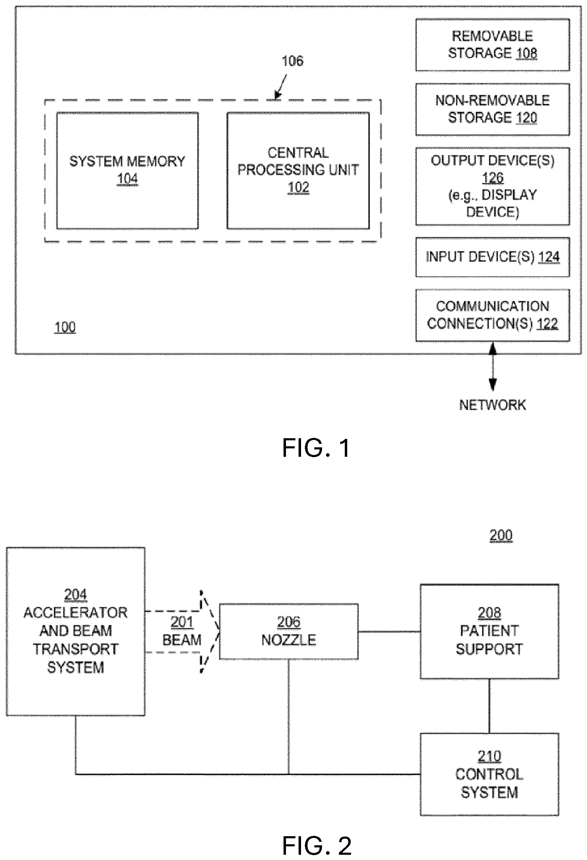

Figure 1 shows a block diagram of an example of a computing system 100 upon which the embodiments described herein may be implemented. In a basic configuration, the system 100 includes at least one processing unit 102 and memory 104. This most basic configuration is illustrated in Figure 1 by dashed line 106. The system 100 may also have additional optional features and/or functionality. For example, the system 100 may also include additional storage (removable and/or non-removable) including, but not limited to, solid state, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included.

In the example of Figure 1, the memory 104 includes computer-readable instructions, data structures, program modules, and the like. Depending on how it is to be used, the system 100 - by executing the appropriate instructions or the like - can be used to implement a planning system used to generate a particle (e.g. proton) therapy treatment plan that employs non-uniform energy layer spacing, as disclosed herein.

Radiation treatment system

Figure 2 is a block diagram showing selected components of a radiation treatment system 200 upon which embodiments according to the present invention can be implemented. In the example of Figure 2, the system 200 includes an accelerator and beam transport system 204 that is operable to generate and/or accelerate a beam 201. The accelerator and beam transport system can generate and deliver beams of various types including, for instance, proton beams, electron beams, ion beams, or atomic nuclei beams (e.g., using elements such as carbon, helium, or lithium). The operations and parameters of the accelerator and beam transport system 204 are controlled so that the intensity, energy, size, and/or shape of the beam are dynamically modulated or controlled during treatment of a patient according to an optimised radiation treatment plan produced by and stored within system 100 as discussed above.

The nozzle 206 is used to aim the beam toward various locations (e.g., of a target) within a patient supported on the patient support device 208 (e.g., a chair, couch, or table) in a treatment room. A target may be an organ, a portion of an organ (e.g., a volume or region within the organ), a tumour, diseased tissue, or a patient outline, for instance.

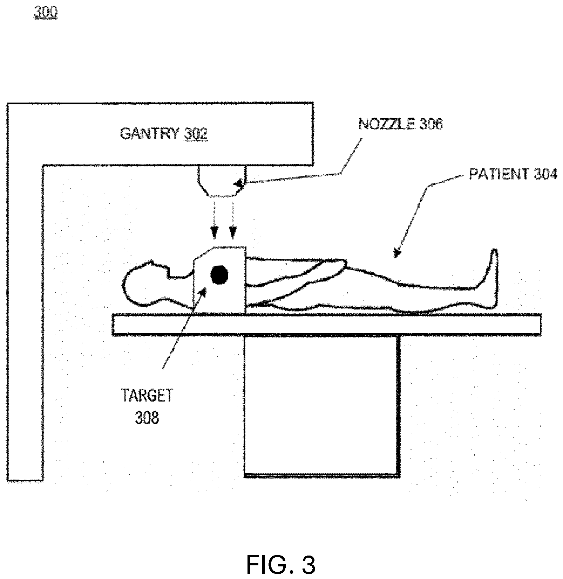

The nozzle 206 may be mounted on or may be a part of a gantry structure (Figure 3) that can be moved relative to the patient support device 208, which may also be moveable. The accelerator and beam transport system 204 may be mounted on or are a part of the gantry structure; alternatively, the accelerator and beam transport system are separate from (but in communication with) the gantry structure.

The control system 210 of Figure 2 receives and implements a prescribed treatment plan which is generated and/or optimised according to embodiments of the present invention. The control system 210 includes a computing system having a processor, memory, an input device (e.g., a keyboard), and optionally a display; the system 100 of Figure 1 is an example of such a platform for the control system 210. The control system 210 can receive data regarding the operation of the system 200. The control system 210 can control parameters of the accelerator and beam transport system 204, nozzle 206, and patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

Figure 3 illustrates exemplary elements of a radiation treatment system 300 for treating a patient 304. The system 300 is an example of an implementation of the radiation treatment system 200 of Figure 2. The gantry 302 may rotate around an axis, e.g. the gantry may rotate around a patient on a couch. The patient may be moved by moving the couch. While the patient 304 is supine in the example of Figure 3, the invention is not so limited. For example, the patient 304 can instead be seated in a chair or positioned in any orientation. The gantry 302 can be controlled by a treatment system using an optimised treatment plan generated according to embodiments of the present invention.

Energy layers

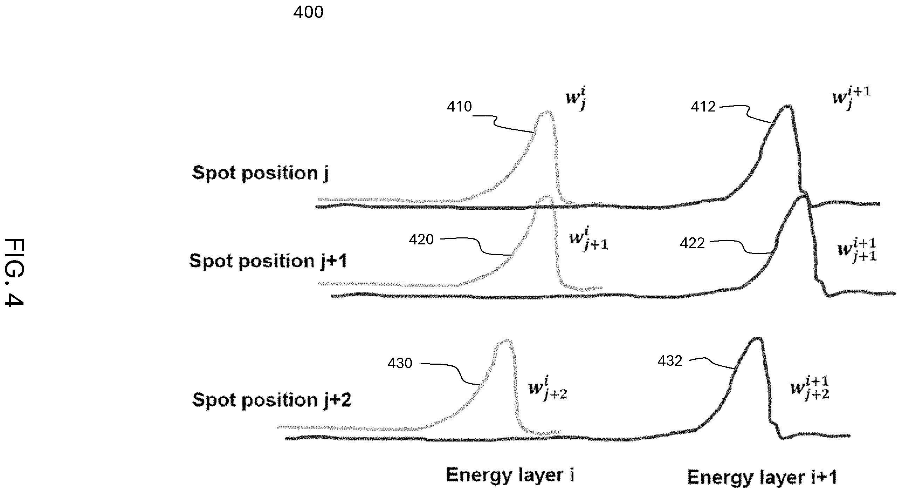

Figure 4 illustrates spot positions and Bragg peak locations of six particle therapy treatment beams. Figure 4 shows three spot positions referred to as "spot position j", "spot position j+1", and "spot position j+2". Also shown are two energy layers, referred to as "Energy layer i" and "Energy layer i+1".

In the present non-limiting example, the spots in each energy layer are each located at the same relative position within the respective energy layer as the relative location of the corresponding spots within the other energy layers. Thus, all energy layers have spots at the same relative positions. So, for example, in Figure 4, Energy layer i is shown as having three spots j, j+1 and j+2, and Energy layer i+1 also has corresponding spots j, j+1 and j+2. Alternatively, spots in different layers are not in the same relative positions in the layers.

Each spot has a corresponding spot weight, and a spot in Energy layer i and at spot position j has a spot weight referred to as $w_j^i$. A spot in Energy layer i+1 at the same relative spot position j but within the energy layer i+1 has a weight of $w_j^{i+1}$. A spot in Energy layer i at spot position j+1 has a weight of $w_{j+1}^i$. A spot in Energy layer i+1 at the same relative spot position j+1 within the energy layer i+1 has a weight of $w_{j+1}^{i+1}$. A spot in Energy layer i at spot position j+2 has a weight of $w_{j+2}^i$. A spot in Energy layer i+1 at the same relative spot position j+2 within the energy layer i+1 has a weight of $w_{j+2}^{i+1}$.

In prior art schemes, the spacing of Energy layers i in Figure 4 is uniform. In contrast, in embodiments according to the present invention, the spacing of Energy layers may be non-uniform.

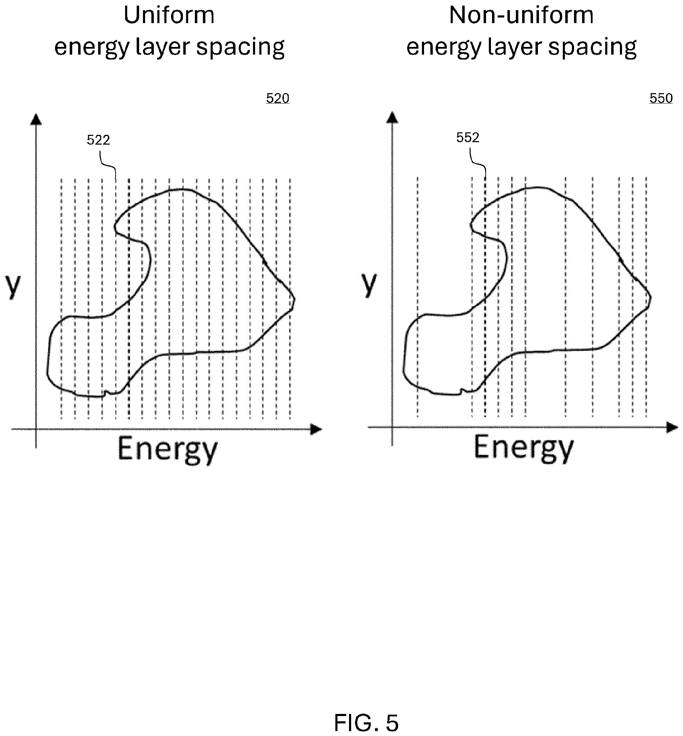

Figure 5 illustrates energy layer spacing. The leftmost graph in Figure 5 shows uniform energy layer spacing 520 as used in the prior art. The rightmost graph in Figure 5 shows non-uniform energy layer spacing 550 in accordance with embodiments of the present invention. While the prior art uniform spacing 520 makes a compromise between dense spacing (leading to complex and time consuming treatment plans) and sparser spacing (leading to inaccurate treatment plans), the non-uniform energy layer spacing 550 of some embodiments of the invention may achieve both (i) a reduction in treatment plan complexity and time, and (ii) increased plan quality and accuracy.

In particular, unimportant layers that are also very close in energy level to their neighbouring layers, may be removed from the particle therapy radiation treatment plan. This is achieved by means of an optimisation scheme that uses an objective function configured to drive the optimisation so that a final outputted treatment plan uses non-uniform energy layer spacing. The optimisation leads to a plan where the energy layer spacing is dense where the dose deposition provided by dense layers is clinically acceptable. Conversely, the optimisation leads to a plan where the energy layer spacing is sparse where the dose deposition provided by sparse layers is clinically acceptable. Thus, the energy layer spacing is dependent on the individual patient geometry, including the locations of tumours or organs at risk in a particular patient.

Further, the shape of the target typically changes from energy layer to energy layer. The optimisation may lead to a plan where the energy layer spacing is relatively dense to deposit dose to a relatively complex target geometry, and relatively sparse to deposit dose to a relatively simple target geometry.

The objective function may be a cost function, and the optimisation may proceed by iteratively modifying the treatment plan parameters so as to minimise the cost of the cost function. Alternatively, the objective function may be a utility function, and the optimisation may proceed by iteratively modifying the treatment plan parameters so as to maximise the utility of the utility function.

*Non-uniformity penalty term*

A non-uniformity penalty term may be introduced in the above-mentioned objective function, such that during optimisation, the non-uniformity penalty term guides the optimisation process to produce treatment plans which have dense energy layer spacing where the dose deposition provided by dense layers is clinically acceptable, and sparser energy layer spacing where the dose deposition provided by sparse layers is clinically acceptable.

In general, in the present specification, the term "non-uniformity penalty term" is used to refer to a penalty term that produces a non-uniform energy layer spacing when used as part of an objective function during treatment plan optimisation.

For example, where a cost function is used, the total cost function, including the non-uniformity penalty term may have a high value if the energy layers or the energy "grid" is relatively sparse in the target. The term "energy grid" is a collective term referring to the energy layers. Further, the total cost function, including the non-uniformity penalty term, may have a high value if the energy layers or the energy grid is relatively dense in an organ at risk. Thus, as such plans are not desirable, they are associated with a higher cost.

Similarly, where a cost function is used, the total cost function, including the non-uniformity penalty term may have a low value if the energy layers or the energy grid is relatively dense in the target. Further, the total cost function, including the non-uniformity penalty term, may have a low value if the energy layers or the energy grid is relatively sparse in an organ at risk. Thus, as such plans are desirable, they are associated with a lower cost.

Where a utility function is used, the total utility function, including the non-uniformity penalty term, may have a low value if the energy layers or the energy grid is relatively sparse in the target. Further, the total utility function, including the non-uniformity penalty term, may have a low value if the energy layers or the energy grid is relatively dense in an organ at risk. Thus, as such plans are not desirable, they are associated with a lower utility.

Similarly, where a utility function is used, the total utility function, including the non-uniformity penalty term may have a high value if the energy layers or the energy grid is relatively dense in the target. Further, the total utility function, including the non-uniformity penalty term, may have a high value if the energy layers or the energy grid is relatively sparse in an organ at risk. Thus, as such plans are desirable, they are associated with a higher utility.

The penalty term may penalise energy layers that are closer in energy level than a threshold energy proximity. This promotes sparser energy layer spacing where this is possible given the clinical goals. The penalty term may penalise spots with an increasing penalty for decreasing spot weights. That is, the lower the spot weight, the higher the penalty. Thus, unimportant spots are more heavily penalised. The penalty term may penalise spots with a constant penalty for spots having a weight lower than a threshold weight. In all these cases, the penalty term has the effect of (i) penalising unimportant spots and (ii) penalising unimportant energy layers.

The penalty term may both (i) penalise energy layers that are closer in energy level than a threshold energy proximity and (ii) penalise spots with an increasing penalty for decreasing spot weights and/or penalises spots with a constant penalty for spots having a weight lower than a threshold weight. This has the effect of "punishing" or penalising unimportant energy layers that are also very close together.

The objective function may comprise a layer penalty term for each energy layer, and the layer penalty term for an energy layer is non-zero only if a sum of weights in the energy layer is lower than a layer sum threshold. Thus, if the total sum of all the spot weights in an energy layer is lower than a layer sum threshold, the energy layer is deemed to be unimportant, and so is penalised.

The sum of all spot weights in an energy layer may be referred to as an energy layer spot weights sum. Each energy layer has a corresponding energy layer spot weights sum. The spot weight sum of the energy layer with the largest spot weights sum is referred to as the maximum spot weights sum. The layer sum threshold may comprise a percentage of the maximum spot weights sum.

The penalty term may be weighted with respect to other terms in the objective function. By varying the weight applied to the penalty term, the extent to which the penalty term guides the optimisation process to achieve non-uniformity in the energy layer spacing may be controlled.

Energy layers in which all spot weights are below a threshold may not be penalised during the optimisation process, but such energy layers may be removed from the treatment plan in a "post-processing" step.

The penalty term may allow the cost of the objective function to occasionally increase when searching for a solution. This increases the probability of the optimiser escaping local minima and increases the probability of finding a globally minimum cost.

The penalty term may be a non-convex function of the spot weights, and may have multiple local minima. In such cases, an optimisation method that allows local minima, such as simulated annealing, may be used.

Method

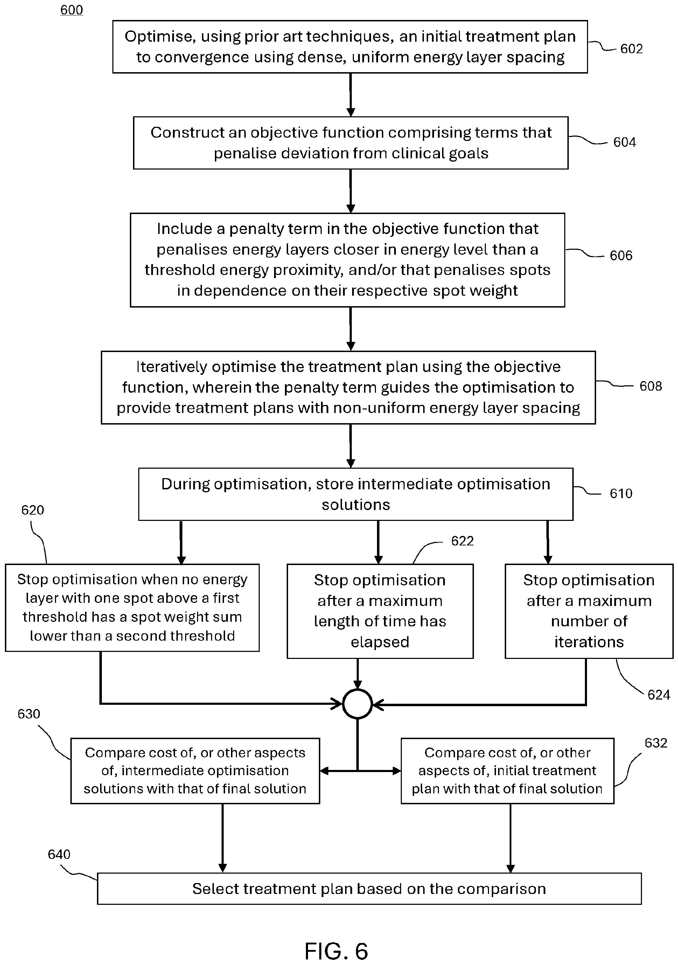

Figure 6 shows a flowchart 600 of a computer-implemented method for optimising a particle therapy treatment plan.

In step 602, an initial treatment plan is optimised to convergence using prior art techniques. The initial treatment plan is optimised to have dense, uniform energy layer spacing. That is, the initial plan (i) has a uniform energy layer spacing (ii) has a small value for the layer spacing, (iii) has fine granularity (iv) leads to more accurate targeting of the tumour. However, such a plan will be complex and the implementation of such a plan will be time consuming.

This optimisation of the initial treatment plan in step 602 will determine spot weights and dose distributions (e.g. dose volume histogram or "DVH" curves), and will achieve, to a certain extent, the clinical goals set for the plan optimisation process. The initial treatment plan is optimised without the use of a penalty term that penalises unimportant close-together energy layers. Thus, the initial treatment plan will disadvantageously have a uniform energy layer spacing. The cost of the initial treatment plan may be calculated using the objective function and then stored for future use. Optionally, one or more of the following types of information may be stored about the initial treatment plan: final spot weights, objective function values for organs at risk and tumours, DVH curves for regions of interest, and information relating to the extent to which clinical objectives or clinical goals are fulfilled.

Advantageously, by starting from a dense energy layer spacing, the optimiser has more freedom with regards to where to position the energy layers of the final output plan. Conversely, if the initial treatment plan already has a relatively sparse energy layer spacing, the optimiser will have less freedom with regards to where to position the energy layers of the final output plan. An initial dense energy layer spacing, by providing more options with regards to which layers to retain and which layers to discard, makes it possible to obtain clinically better solutions.

In step 604, an objective function is constructed to further optimise the initial treatment plan. The objective function may take the form of either a cost function or a utility function, and may comprise terms that are designed to penalise deviations from clinical goals, or comprise terms that are designed to reward adherence to clinical goals. For example, goals may specify a minimum or maximum dose to be applied to a tumour or an OAR. As further examples, goals may specify the required dose homogeneity in an organ, or the mean dose to be delivered to a region of interest.

In step 606, a non-uniformity penalty term is included in the objective function that penalises energy layers that are closer in energy level and/or penalises spots in dependence on their respective spot weights. Low-weight spots are considered to be less important than high-weight spots. Thus, the non-uniformity penalty term has the effect of penalising less important and close-together energy layers. This leads to a treatment plan with non-uniform energy layer spacing, wherein no or only sparse energy layers are applied where the dose deposition provided by sparse layers is clinically acceptable. Conversely, dense energy layers are applied where the dose deposition provided by dense layers is clinically acceptable.

In step 608, the objective function including the non-uniformity penalty term is iteratively optimised in accordance with known techniques to produce a treatment plan with non-uniform energy layer spacing.

During optimisation, in step 610, the results of each iteration are stored as one or more intermediate optimisation solutions. The results of an iteration may be stored for one, some, or all future iterations. If one or more subsequent iterations result in an improved solution, the previous iterations may be discarded. If an iteration results in the finding of a local minimum, advantageously the iteration results may be stored until the end of the optimisation, or even after the end of the optimisation, such as permanently. For example, when an iteration is stored, information including spot weights, objective function values for organs at risk and tumours, DVH curves for regions of interest, and the extent to which clinical objectives or clinical goals are fulfilled may be stored.

The spot weights in each energy layer may be modified in each iteration.

The optimisation process may end in a number of ways. Three exemplary ways of ending the optimisation are illustrated by step 620, step 622 and step 624.

In step 620, the optimisation is stopped when no energy layer with one spot above a first threshold has a spot weight sum lower than a second threshold. Alternatively, the optimisation may be stopped when no energy layer with one spot above the first threshold has a sum of squared spot weights lower than a third threshold. All energy layers with no spot above the first threshold are removed (for instance, in a post-processing stage).

In step 622, the optimisation is stopped after a maximum length of time has elapsed.

In step 624, the optimisation is stopped after a maximum number of iterations.

In general, the optimisation may be stopped after one or more of the requirements specified in step 620, 622 and 624 are met. The treatment plan when the process stops at step 620, 622 and 624 is the final treatment solution provided by the iterative optimisation step 608.

In a post-processing step, if there are any energy layers that are found to be empty after step 620, 622 or 624, these may be discarded from the treatment plan.

Once the optimisation is finished, the cost of each of the intermediate solutions is compared with the cost of the final treatment solution, in step 630. Optionally, the following aspects of the intermediate solutions and the final treatment solution may be compared: objective function values for organs at risk and tumours, DVH curves for regions of interest, and the extent to which each treatment plan fulfils the clinical objectives or clinical goals.

Alternatively or additionally, once the optimisation is finished, the cost of the initial treatment plan is compared with the cost of the final treatment solution, in step 632. Optionally, the following aspects of the initial treatment plan and the final treatment solution may be compared: objective function values for organs at risk and tumours, DVH curves for regions of interest, and the extent to which the initial treatment plan fulfils the clinical objectives or clinical goals. By comparing the cost of the initial treatment plan with the cost of the final solution, the dense uniform energy layer spacing of the prior art is compared to the optimal non-uniform energy layer spacing produced by embodiments of the present invention.

The final dose of any two of the initial, intermediate and final treatment solutions may also be compared to establish the extent to which each solution provides an ideal dose, or identify the solution which provides the dose closest to the ideal dose.

In step 640, a treatment plan is selected based on the comparisons performed in step 630 and/or step 632.

The comparisons of the initial, intermediate and final treatment solutions as performed in step 630 and/or 632 may be performed by a computer. The selection of the treatment plan in step 640 may also be performed by a computer. For example, the lowest cost treatment plan may be automatically selected. Alternatively, a human user may choose between the initial, final or intermediate solutions, which will each have different energy layer spacings.

Due to the inclusion of the non-uniformity penalty term in the objective function, the optimised treatment plan produced using the flowchart 600 of Figure 6 has a non-uniform energy layer spacing with dense spacing where clinically acceptable, and sparse spacing where clinically acceptable. This reduces delivery time and complexity where possible whilst maintaining treatment accuracy where needed.

The optimisation of Figure 6 allows the energy layer spacing of a treatment plan to be tailored to an individual patient's geometry. Patients with different geometry are irradiated in accordance with treatment plans such that regions of a specific patient that require treatment are irradiated with dense energy layer spacings, and regions of the specific patient that require less treatment are irradiated with sparse energy layer spacings.

As the above method is used to determine which energy layers to retain and which energy layers to discard, it is effectively regulating the proton beam, and may therefore be seen as a form of beamline calculation.

Examples of objective functions and penalty terms

Some examples of objective functions and non-uniformity penalty terms as described above are now provided. It is to be understood that these examples are non-limiting, and in practice there may be any number of alternatives or variations in the equations describing the objective function and/or the non-uniformity penalty term.

*Example #1*

A typical objective function for optimisation of a radiation therapy treatment plan is the following cost function:

$$\text{minimise}_{\{w_j^i\}} \left( C(\{w_j^i\}) \right)$$

Equation 1

where

C = cost function with objectives (e.g. goal doses for the tumour and/or OAR)
$w_j^i$ = weight of spot j in energy layer i ($\geq 0$)
$\{w_j^i\}$ = the set of all spot weights of all the spots j across all the layers i
i = energy layer index
j = spot index

The cost function may penalise deviations from clinical goals such as, for example, a clinical goal specifying a minimum dose to a tumour, or a maximum dose to an organ at risk. Generally, the aim of the optimisation is to find a set of treatment parameters that minimise the cost function C. In the example of Equation 1, the aim of the optimisation is to find a set of weights $\{w_j^i\}$ that minimise the cost function C. Spot weights are always larger than or equal to zero, and in principle have no upper bound.

In the optimisation of the above objective function, the treatment parameters and spot weights are modified, the cost is re-calculated, and this is repeated with an aim to minimising cost. That is, the treatment parameters or spot weights are modified iteratively to reduce the cost of the objective function.

*Example #2*

Energy layers may be suppressed by including a non-uniformity penalty term P in the above prior art cost function of Equation 1:

$$\text{minimise}_{\{w_j^i\}} \left( C(\{w_j^i\}) + \lambda \cdot P(\{w_j^i\}) \right)$$

Equation 2

where

C = cost function with objectives (e.g. goal doses for PTV and OAR)
P = penalty term
$\lambda$ = weight of penalty term
$w_j^i$ = weight of spot j in energy layer i ($\geq 0$)
$\{w_j^i\}$ = the set of all spot weights of all the spots j across all the layers i
i = energy layer index
j = spot index

In the above equation, C and P are functions with values greater than or equal to zero. Specifically, P comprises a penalty term. The penalty term may be configured to penalise unimportant spots in close energy layers, leading to non-uniform energy layer spacing.

The variable $\lambda$ weights the penalty term P in relation to the rest of the cost or objective function, and may comprise a positive constant. A larger value of $\lambda$ increases the possibility of discarding spots to achieve non-uniform energy layer spacing. Conversely, a smaller value of $\lambda$ decreases the possibility of discarding spots to achieve non-uniform energy layer spacing. Parameter $\lambda$ may be inputted by a human user. Alternatively, $\lambda$ may be determined automatically to give a penalisation that is a certain percentage of the prior art uniform layer cost function C. Thus, objective functions with a low $\lambda$ would retain low-weight spots to a greater extent than objective functions with a higher $\lambda$. Thus, by controlling the value of $\lambda$, layers with small weights may be retained if this is required by the overall clinical objectives, and/or layers with small weights may be discarded if this is allowed by the overall clinical objectives.

*Example #3*

An alternative objective function is as follows:

$$\text{minimise}_{\{w_j^i\}} \left( C(\{w_j^i\}) + \lambda \cdot P(\{w_j^i\}, \{E^i\}) \right)$$

Equation 3

where

C = cost function $\geq 0$
P = penalty function $\geq 0$
$\{w_j^i\}$ = the set of all spot weights of all the spots j across all the layers i
$\lambda$ = positive constant
$\{E'\}$ = set of all energy layers
i = energy layer index
j = spot index

In this example, the penalty term is not only a function of the set of spot weights, but also a function of the set of energy layers. By means of this equation, less important energy layers may be suppressed. Optimising this objective function would result in a treatment plan with optimal non-uniform energy layer spacing, with unimportant and close-together energy layers being discarded, and the important spread-out energy layers being retained.

*Example #4*

A specific example of a non-uniformity penalty term usable in the above objective functions may be written as:

$$P(\{w_j^i\}) = \sum_{i=1}^{N_E} \left[ \frac{Q_i}{1 + \sum_{j=1}^{N_s} R(\{w_j^i\})} \right]$$

where:

$$R(w_j^i) = \begin{cases} \left(w_j^i - w_{\min MU}\right)^k, & \text{if } w_j^i > w_{\min MU} \\ 0, & \text{else} \end{cases}$$

and where:

$$Q_i = \begin{cases} 0 \text{ if } \sum_{j=1}^{N_s} w_j^i > threshold \text{ OR } w_j^i < \varepsilon \text{ for all spot positions j} \\ 1, else \end{cases}$$

Equation 4

where

P = penalty term
$\{w_j^i\}$ = the set of all spot weights of all the spots j across all the layers i
$N_E$ = number of energy layers
Ns = number of spots
$Q_i$ = small spot weight sum layer penalty
R = penalises spots close to or below $w_{minMU}$
$w_j^i$ = weight of spot j in energy layer i ($\geq 0$)
$w_{minMU}$ = spot weight when the radiation therapy system provides minimum monitor units (MUs)
i = energy layer index
j = spot index
k = emphasises low spot weight penalty, constant $\geq 1$

The *threshold* in Equation 4 may be a percentage of the maximum spot weights sum of the energy layers. The term $w_{minMU}$ refers to the spot weight corresponding to the minimum monitor units that can be provided by the radiation therapy system. Spots below this threshold are removed during the optimisation or during postprocessing.

By means of the Q term, only energy layers with a small spot weight sum are penalised. $Q_i$ also does not penalise energy layers where all the spots are lower than a threshold $\varepsilon$. Such layers are more easily removed during a post-processing stage.

The *R* term is zero when the spot weight is lower than $w_{minMU}$. The *R* term is non-zero but has a small value when the spot weight is just above $w_{minMU}$. Low values of *R* result in a higher penalty. Also, by means of the *R* term, if the energy layer has one or more sufficiently large spots, the penalty drops to a small value. This has the effect of penalising important layers to a lesser extent than unimportant layers.

The variable "k" may be selected to control the penalty applied to spots with a weight above $w_{minMU}$. The most appropriate value for "k" is determined empirically by trial and error.

*Example #5*

A further, alternative example of a penalty term is:

$$P\left(\{w_j^i\}, \{E^i\}\right) = \sum_{i=1}^{N_E} \left[ \frac{aQ_i}{1 + c\sum_{j=1}^{N_s} R\left(w_j^i\right)} + \frac{bS_i}{|[\Delta E]_i^i|} \right]$$

where

$$R\left(w_j^i\right) = \begin{cases} \left(w_j^i - w_{minMU}\right)^k, & \text{if } w_j^i > w_{minMU}, \\ 0, & \text{else}, \end{cases}$$

$$Q_i = \begin{cases} 0, & \text{if } \overline{w}^i = \sum_{j=1}^{N_s} w_j^i > p \cdot \max_i \overline{w}^i \text{ or } w_j^i < \varepsilon \text{ for all spot positions } j \\ 1, & \text{else}. \end{cases}$$

$$S_i = \begin{cases} 1 \text{ if at least one spot weight of layer } i > \varepsilon \\ 0, \text{else} \end{cases}$$

Equation 5

where

P = penalty term
$\{w_j^i\}$ = the set of all spot weights of all the spots j across all the layers i
$\{E^i\}$ = the set of all energy layers

$|[\Delta E]_i|$ = energy interval (see explanation below)
$S_i$ = minimum spot weight in layer i greater than $\varepsilon$
$N_E$ = number of energy layers
$N_s$ = number of spots
$Q_i$ = small spot weight sum layer penalty (see also discussion below)
$R$ = penalises spots close to or below $w_{minMU}$
$\underline{w^j}$ = weight of spot j in energy layer i ($\geq 0$)
$\overline{w^i}$ = sum of spot weights in layer i
p = percentage of maximum spot sum across energy layers
$\varepsilon$ = reduces penalty if all spots in a layer are below this threshold
$w_{minMU}$ = spot weight when the radiation therapy system provides minimum monitor units (MUs)
i = energy layer index
j = spot index
k = controls low spot weight penalty, constant $\geq 1$
a, b, c = constants (see explanation below)
l = emphasises energy interval, constant determined empirically

In the above equations, $|[\Delta E]_i|$ is the absolute value of the energy interval between the energy layer *i* and its closest neighbouring energy layer with spot weights above $w_{minMU}$. The term $Q_i$ in the above equation ensures that only energy layers with a small, non-zero spot weight sum are penalised. The reason for penalising such layers is because due to their low spot weight sum they are less important than other layers, and also because removal of such layers is more easily compensated for by increasing the weight of spots in neighbouring layers.

$Q_i$ also does not penalise energy layers where all the spots are lower than a threshold $\varepsilon$. Such layers are more easily removed during a post-processing stage. Similarly, spots with a weight below $w_{minMU}$ may be removed during optimisation or during postprocessing.

The R term is zero when the spot weight is lower than $w_{minMU}$. The *R* term is non-zero but has a small value when the spot weight is just above $w_{minMU}$. Low values of *R* result in a higher penalty. If a layer has one or more sufficiently large spots, the penalty for that specific layer drops to a small value (that is, the R term in the above equation becomes large). In such cases, it is inferred that due to the large spot weights, that energy layer is important for fulfilling the clinical goals and objectives, and so they should not be "punished" or penalised.

As the spot weights in the radiation therapy plan evolve during the optimisation process, the layers that have a small sum of spot weights changes. That is, some energy layers may evolve to having a small spot weight sum whilst other energy layers may evolve to having a large spot weight sum. Thus, the layers that are punished or penalised for having a small spot weight sum evolves over the optimisation process.

The second term of the above equation (i.e., with numerator $bS_i$ and denominator $|[\Delta E]_i|^l$) guides the optimisation process so that the energy layers that are close together are less "attractive" and therefore penalised more. This discourages solutions with dense energy layer spacing. The term $S_i$ ensures that only layers with one spot above a threshold $\varepsilon$ are penalised. Layers with no spots above a threshold $\varepsilon$ are removed during a post-processing step.

The parameters *a*, *b* and *c* of the above equation may be modified to emphasise different parts of the penalty term. These parameters may generally be determined empirically. That is, the values of *a*, *b* and *c* which work in practice are determined empirically before optimisation and then subsequently used during the optimisation process.

*Example #6*

A still further example of a penalty term is:

$$P\left(\{w_j^i\}, \{E^i\}\right) = \sum_{i=1}^{N_E} \frac{Q_i}{\left(1 + \sum_{j=1}^{N_s} R\left(w_j^i\right)\right) |[\Delta E]_i|^l}$$

Equation 6

where

P = penalty term
$\{w_j^i\}$ = the set of all spot weights of all the spots j across all the layers i
$\{E^i\}$ = the set of all energy layers

$|[\Delta E]_i|$ = energy interval (see explanation above)

$N_E$ = number of energy layers

Ns = number of spots

$Q_i$ = small spot weight sum layer penalty (see Example #5)

R = penalises spots close to or below $w_{minMU}$ (see Example #5)

$w_j^i$ = weight of spot j in energy layer i ($\geq 0$)

i = energy layer index

j = spot index

I = emphasises energy interval, constant determined empirically, $\geq 1$

In this example, again, the factor $|[\Delta E]_i|$ would guide the optimisation process to penalise energy layers that are too close to their nearest neighbour, and the function R would penalise spots with small weights. The functions R and Q may be as defined above for Example #5. The loss of dose contribution from spots or energy layers that are penalised and removed from the treatment plan may be compensated for by increasing the weights of the closest retained neighbouring spots or energy layers.

*Example #7*

A further example of a penalty term is:

$$P\left(\{w_j^i\}, \{E^i\}\right) = \sum_{i=1}^{N_E} \left[ \frac{aQ_i}{1 + c\sum_{j=1}^{N_s} R(w_j^i)} + \frac{bS_i}{|[\Delta E]_i|^I} \right]$$

where

$$R(w_j^i) = \begin{cases} \left(w_j^i - w_{\min MU}\right)^k, & \text{if } w_j^i > w_{\min MU}, \\ 0, & \text{else}, \end{cases}$$

$$Q = \begin{cases} 0 \; if \; \sum_{j=1}^{N_s} \left(w_j^i\right)^2 > p \; OR \; w_j^i < \varepsilon \; \text{for all spot positions j} \\ 1, else \end{cases}$$

Equation 7

where the variables of Equation 7 are the same as in Equation 5, except for Q.

That is, Equation 7 is the same as Equation 5, except with a different formula for Q. In particular, instead of defining Q in relation to the sum of spot weights as in Equation 5, Q of Equation 7 is defined in relation to the sum of squared spot weights. Apart from this difference, Equation 7 works the same way as Equation 5. The different formula for Q results in a different distribution of spot weights.

Any of the above Equations 1-7 may be used as penalty terms in an objective function to produce non-uniform energy layer spacing oriented towards achieving both an accurate treatment plan that is also not too time consuming or complex. Empirical investigations may be performed to establish which of the above equations should be used in a wide variety of real-world clinical cases.

Optimisation schemes

The above described cost functions may be optimised using any of a number of known optimisation schemes, including for example, simulated annealing, genetic algorithms, or the Limited-Memory Broyden-Fletcher-Goldfarb-Shanno (LBFGS) algorithm. Any optimisation algorithm suitable for non-linear optimisation may be used. As these optimisation schemes are understood by the skilled person, they need no elaboration here.

The key principle is that a novel non-uniformity penalty term may be used in an objective function for an optimisation scheme to provide treatment plans with non-uniform energy layer spacing to improve the trade-off between treatment accuracy and treatment complexity.

Embodiments of the present invention are thus described. While the present invention has been described in particular embodiments, it should be appreciated that the present invention should not be construed as limited by such embodiments, but rather construed according to the following claims.

**Claims**

1. A method of computer-assisted particle therapy treatment planning comprising generating a treatment plan that uses non-uniform energy layer spacing.

2. The method of claim 1 further comprising optimising the treatment plan using an objective function that permits non-uniform energy layer spacing in the generated treatment plan.

3. The method of claim 2, wherein the objective function comprises a cost function with terms that penalise deviation from clinical goals, and wherein said optimisation comprises minimising the cost function; and
wherein the objective function further contains a penalty term that guides the optimisation of the treatment plan to provide non-uniform energy layer spacing in the generated treatment plan.

4. The method of claim 3, wherein the penalty term penalises energy layers that are closer in energy level than a threshold energy proximity.

5. The method of claim 3, wherein the penalty term penalises spots with an increasing penalty for decreasing spot weights, and/or penalises spots with a constant penalty for spots having a weight lower than a threshold weight.

6. The method of claim 3, wherein the penalty term both (i) penalises energy layers that are closer in energy level than a threshold energy proximity and (ii) penalises spots with an increasing penalty for decreasing spot weights and/or penalises spots with a constant penalty for spots having a weight lower than a threshold weight.

7. The method of any of claim 2 to claim 6, wherein the objective function comprises a layer penalty term for each energy layer, and wherein the layer penalty term for an energy layer is non-zero only if a sum of spot weights in the energy layer is lower than a layer sum threshold.

8. The method of claim 7, wherein:

   the sum of all spot weights in each energy layer comprises an energy layer spot weights sum;
   the spot weights sum of the energy layer with the largest spot weights sum comprises a maximum spot weights sum; and
   the layer sum threshold comprises a percentage of the maximum spot weights sum.

9. The method of any of claim 3 to claim 8, further comprising weighting the penalty term with respect to other terms in the objective function to control an extent of non-uniformity in the energy layer spacing.

10. The method of any of claim 2 to claim 9, comprising:

    optimising an initial treatment plan until convergence using uniform energy layer spacing, wherein optimisation of the initial treatment plan does not use a penalty term that guides the optimisation to non-uniform energy layer spacing;
    iteratively applying the objective function to the initial treatment plan and to successive intermediate treatment plans to optimise and obtain a final treatment plan that uses non-uniform energy layer spacing.

11. The method of claim 10, further comprising stopping the iterative optimisation when none of the energy layers with at least one spot weight above a global spot weight threshold, have a spot weight sum lower than an iteration stop spot weight sum threshold;
    and/or:
    the method further comprising stopping the iterative optimisation after a threshold length of time, and/or a threshold number of iterations, have lapsed since a start of the optimisation.

12. The method of claim 10 or claim 11, further comprising:

during optimisation, storing one or more intermediate treatment plans;
calculating a cost of each of the intermediate treatment plans;
at the end of the optimisation, comparing each intermediate treatment plan to the final treatment plan; and
selecting between the intermediate treatment plans and the final treatment plan in dependence on a plan that has
a lowest cost;
and/or:
wherein the method comprises:

calculating a cost of the initial treatment plan that uses uniform energy layer spacing;
at the end of the optimisation, comparing the initial treatment plan to the final treatment plan; and
selecting between the initial treatment plan and the final treatment plan in dependence on a plan that has a
lowest cost.

13. The method of any of claim 3 to claim 12, wherein the penalty term is of the form:

$$P\left(\{w_j^i\}, \{E^i\}\right) = \sum_{i=1}^{N_E} \left[ \frac{aQ_i}{1 + c\sum_{j=1}^{N_s} R\left(w_j^i\right)} + \frac{bS_i}{|[\Delta E]_i|^l} \right]$$

where

$$R\left(w_j^i\right) = \begin{cases} \left(w_j^i - w_{\min MU}\right)^k, & \text{if } w_j^i > w_{\min MU}, \\ 0, & \text{else,} \end{cases}$$

$$Q_i = \begin{cases} 0, & \text{if } \overline{w}^i = \sum_{j=1}^{N_s} w_j^i > p \cdot \max_i \overline{w}^i \text{ or } w_j^i < \varepsilon \text{ for all spot positions } j \\ 1, & \text{else.} \end{cases}$$

$$S_i = \begin{cases} 1 \text{ if at least one spot weight of layer } i > \varepsilon \\ 0, \text{otherwise} \end{cases}$$

where

P = penalty term
$\{w_j^i\}$ = the set of all spot weights of all the spots j across all the layers i
$\{E^i\}$ = the set of all energy layers
$|[\Delta E]_i|$ = energy interval
$S_i$ = minimum spot weight in layer i greater than $\varepsilon$
$N_E$ = number of energy layers
Ns = number of spots
$Q_i$ = small spot weight sum layer penalty
R = penalises spots close to or below $w_{minMU}$
$\underline{w_j^i}$ = weight of spot j in energy layer i ($\geq 0$)
$\overline{w}^i$ = sum of spot weights in layer i
p = percentage of maximum spot sum across energy layers
$\varepsilon$ = reduces penalty if all spots in a layer are below this threshold
$w_{minMU}$ = spot weight when the radiation therapy system provides minimum monitor units
i = energy layer index
j = spot index
k = controls low spot weight penalty, constant $\geq 1$
a, b, c = constants
l = emphasises energy interval, constant determined empirically

**14.** A radiation treatment planning computer system comprising a memory and a processor configured to perform the method as defined in any one of claims 1-13.

**15.** A computer program product comprising a non-transitory, computer readable storage medium encoded with instructions operable for execution by a processor to perform the method as defined in any one of claims 1-13.

FIG. 1

FIG. 2

FIG. 3

FIG. 4

## Uniform
### energy layer spacing

520

522

y

Energy

## Non-uniform
### energy layer spacing

550

552

y

Energy

FIG. 5

600

| Optimise, using prior art techniques, an initial treatment plan to convergence using dense, uniform energy layer spacing | 602 |

↓

| Construct an objective function comprising terms that penalise deviation from clinical goals | 604 |

↓

| Include a penalty term in the objective function that penalises energy layers closer in energy level than a threshold energy proximity, and/or that penalises spots in dependence on their respective spot weight | 606 |

↓

| Iteratively optimise the treatment plan using the objective function, wherein the penalty term guides the optimisation to provide treatment plans with non-uniform energy layer spacing | 608 |

↓

| During optimisation, store intermediate optimisation solutions | 610 |

620

| Stop optimisation when no energy layer with one spot above a first threshold has a spot weight sum lower than a second threshold |

622

| Stop optimisation after a maximum length of time has elapsed |

| Stop optimisation after a maximum number of iterations |

624

630

| Compare cost of, or other aspects of, intermediate optimisation solutions with that of final solution |

632

| Compare cost of, or other aspects of, initial treatment plan with that of final solution |

640

| Select treatment plan based on the comparison |

FIG. 6

Europäisches
Patentamt

European
Patent Office

Office européen
des brevets

**EUROPEAN SEARCH REPORT**

Application Number

EP 24 19 4359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/308485 A1 (KOPONEN TIMO [FI] ET AL) 7 October 2021 (2021-10-07) * the whole document * | 1,2,14, 15 | INV. A61N5/10 |
| X | US 2010/327188 A1 (BERT CHRISTOPH [DE] ET AL) 30 December 2010 (2010-12-30) * paragraph [0046] - paragraph [0062] * | 1,2,14, 15 | |
| X | US 2022/233883 A1 (ANDERSSON BJORN [SE] ET AL) 28 July 2022 (2022-07-28) * the whole document * | 1-8, 10-15 | |
| Y | | 9 | |
| X | JANSON MARTIN ET AL: "Treatment planning of scanned proton beams in RayStation", MEDICAL DOSIMETRY, vol. 49, no. 1, 22 November 2023 (2023-11-22), pages 2-12, XP093235212, US ISSN: 0958-3947, DOI: 10.1016/j.meddos.2023.10.009 * the whole document * | 1,2,14, 15 | |
| | | | **TECHNICAL FIELDS SEARCHED (IPC)** |
| | | | A61N |
| X | ALSHAIKHI JAILAN ET AL: "Impact of varying planning parameters on proton pencil beam scanning dose distributions in four commercial treatment planning systems", MEDICAL PHYSICS., vol. 46, no. 3, 1 February 2019 (2019-02-01), pages 1150-1162, XP093235218, US ISSN: 0094-2405, DOI: 10.1002/mp.13382 Retrieved from the Internet: URL:https://api.wiley.com/onlinelibrary/td m/v1/articles/10.1002%2Fmp.13382> * the whole document * | 1,2,14, 15 | |

-/--

The present search report has been drawn up for all claims

| Place of search | Date of completion of the search | Examiner |
|---|---|---|
| Munich | 18 December 2024 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                      
& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 19 4359

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/091488 A1 (DING XUANFENG [US] ET AL) 28 March 2019 (2019-03-28) * the whole document * | 1-8, 10-15 | |
| Y | US 2023/310888 A1 (LANSONNEUR PIERRE [FI] ET AL) 5 October 2023 (2023-10-05) * paragraph [0054] * | 9 | |
| A | US 2021/154492 A1 (ENGWALL ERIK [SE] ET AL) 27 May 2021 (2021-05-27) * the whole document * | 1-15 | |

**TECHNICAL FIELDS SEARCHED (IPC)**

The present search report has been drawn up for all claims

| Place of search | Date of completion of the search | Examiner |
|---|---|---|
| Munich | 18 December 2024 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 4359

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2024

| Patent document cited in search report | | Publication date | Patent family member(s) | | Publication date |
|---|---|---|---|---|---|
| US 2021308485 | A1 | 07-10-2021 | CN | 115551589 A | 30-12-2022 |
| | | | EP | 4126215 A1 | 08-02-2023 |
| | | | US | 2021308485 A1 | 07-10-2021 |
| | | | WO | 2021198197 A1 | 07-10-2021 |
| US 2010327188 | A1 | 30-12-2010 | DE | 102008009765 A1 | 03-09-2009 |
| | | | EP | 2279027 A1 | 02-02-2011 |
| | | | US | 2010327188 A1 | 30-12-2010 |
| | | | WO | 2009103467 A1 | 27-08-2009 |
| US 2022233883 | A1 | 28-07-2022 | CN | 113840631 A | 24-12-2021 |
| | | | EP | 3750596 A1 | 16-12-2020 |
| | | | JP | 2022538756 A | 06-09-2022 |
| | | | US | 2022233883 A1 | 28-07-2022 |
| | | | WO | 2020249418 A1 | 17-12-2020 |
| US 2019091488 | A1 | 28-03-2019 | CN | 109195663 A | 11-01-2019 |
| | | | EP | 3426346 A1 | 16-01-2019 |
| | | | JP | 7475810 B2 | 30-04-2024 |
| | | | JP | 2019507658 A | 22-03-2019 |
| | | | JP | 2023014092 A | 26-01-2023 |
| | | | JP | 2024107009 A | 08-08-2024 |
| | | | US | 2019091488 A1 | 28-03-2019 |
| | | | WO | 2017156419 A1 | 14-09-2017 |
| US 2023310888 | A1 | 05-10-2023 | CN | 116920285 A | 24-10-2023 |
| | | | EP | 4252840 A1 | 04-10-2023 |
| | | | US | 2023310888 A1 | 05-10-2023 |
| US 2021154492 | A1 | 27-05-2021 | CN | 110636883 A | 31-12-2019 |
| | | | EP | 3421087 A1 | 02-01-2019 |
| | | | EP | 3645113 A1 | 06-05-2020 |
| | | | JP | 6878626 B2 | 26-05-2021 |
| | | | JP | 2020523118 A | 06-08-2020 |
| | | | US | 2021154492 A1 | 27-05-2021 |
| | | | WO | 2019002463 A1 | 03-01-2019 |

EPO FORM P0459

For more details about this annex : see Official Journal of the European Patent Office, No. 12/82